# EUROPÄISCHE PATENTSCHRIFT

(11) **EP 0 693 114 B1**
(45) Veröffentlichungstag und Bekanntmachung des Hinweises auf die Patenterteilung: **14.05.1997**
(21) Anmeldenummer: 94913068.6
(22) Anmeldetag: 28.03.1994
(51) Int. Cl.: C11D 1/825, C11D 1/94, A61K 7/50

(54) **HANDGESCHIRRSPÜLMITTEL**
MANUAL DISHWASHING COMPOSITION
COMPOSITION POUR LAVER LA VAISSELLE A LA MAIN

(30) Priorität: 05.04.1993 DE 4311114
(43) Veröffentlichungstag der Anmeldung: 24.01.1996
(73) Patentinhaber: Henkel Kommanditgesellschaft auf Aktien, 40191 Düsseldorf (DE)
(72) Erfinder: SCHMID, Karl, D-40822 Mettmann (DE); GIESEN, Brigitte, D-40235 Düsseldorf (DE); KOREN, Karin, D-40227 Düsseldorf (DE)
(86) Internationale Anmeldenummer: EP9400975
(87) Internationale Veröffentlichungsnummer: WO9422997

(56) Entgegenhaltungen:
- EP-A- 0 408 965
- WO-A-91/14760
- WO-A-92/20768
- DE-A- 4 124 247
- DE-A- 4 237 178
- GB-A- 2 242 686
- US-A- 4 240 921

## Beschreibung

### Gebiet der Erfindung

Gegenstand der Erfindung sind wäßrige Handgeschirrspülmittel, enthaltend Alkyl- und/oder Alkenyloligoglykoside, ausgewählte Fettalkoholpolyglycolether und amphotere bzw. zwitterionische Tenside.

### Stand der Technik

Alkyloligoglykoside und insbesondere Alkyloligoglucoside stellen nichtionische Tenside dar, die wegen ihrer nativen Rohstoffbasis - Fettalkohol und Zucker - zunehmend an Bedeutung gewinnen und beispielsweise in manuellen Spülmitteln oder kosmetischen Produkten eingesetzt werden [vgl. **Tens. Surf.Det. 28, 413 (1991)**]. Trotz guter anwendungstechnischer Ergebnisse besteht dennoch das Bedürfnis nach Detergensmischungen auf Basis von Alkylglucosiden, deren Leistungsvermögen das der Einzelstoffe in synergistischer Weise übersteigt.

In der Vergangenheit hat es nicht an Versuchen gemangelt, Detergensgemische auf Basis von Alkyloligoglucosiden zu entwickeln, die über vorteilhafte Eigenschaften verfügen.

Aus den Patentschriften **EP-B-0 070 074**, **EP-B-0 070 075**, **EP-B-070 076** und **EP-B-0 070 077** (Procter & Gamble) sind beispielsweise schaumstarke Kombinationen von Alkyloligoglucosiden mit anionischen Tensiden, wie Seifen, Alkylbenzolsulfonaten, Fettalkoholsulfaten, konventionellen Fettalkoholethersulfaten, alpha-Olefinsulfonaten, Alkansulfonaten sowie gegebenenfalls Betaintensiden bekannt.

Gemäß der Lehre der Patentschrift **EP-B1 0 075 996** (Procter & Gamble) können wäßrige Reinigungsmittel Alkylpolyglucoside, Fettalkoholpolyglycolether und Detergensgerüststoffe enthalten. Eine ähnliche Zusammensetzung wird auch in der Deutschen Offenlegungsschrift **DE-A1 40 39 223** (Hüls) beansprucht.

In der Deutschen Offenlegungsschrift **DE-A1 35 34 082** werden ferner hautfreundliche Geschirrspülmittel vorgeschlagen, die eine Kombination anionischer Sulfat- bzw. Sulfonattenside, Alkyloligoglucoside und Fettsäurealkanolamide enthalten.

Gegenstand der Britischen Offenlegungsschrift **GB-A 22 42 686** sind Detergensgemische für die Haut- und Körperreinigung, die (a) Anlagerungsprodukte von durchschnittlich 1 bis 20 Mol Propylenoxid und 0 bis 30 Mol Ethylenoxid an aliphatische Alkohole mit 1 bis 8 Kohlenstoffatomen, (b) Alkyloligoglucoside und gegebenenfalls (c) amphotere Tenside enthalten. Auch aus der **EP-A 0 408 965** sind Detergensgemische bekannt, die Alkyloligoglucoside und ausgewählte Fettalkoholpolyglycolether enthalten. In der **WO 92/20768** wird die Verwendung von Dialkylethern, gegebenenfalls gemeinsam mit Fettalkoholpolyglycolethern und Alkyloligoglucosiden als Schaumregulatoren für schaumarme Reinigungsmittel vorgeschlagen. Die **DE-A 41 24 247** befaßt sich mit der Stabilisierung wäßriger Zeolith-Suspensionen durch Zusatz eines Tensidgemisches aus Alkyloligoglucosiden und bestimmten Fettalkoholpolyglycolethern. In der **US 4,240,921** werden schaumgedämpfte Zusammensetzungen zum Reinigen von Flaschen vorgeschlagen, die Fettalkoholpropylenglycol/ethylenglycolether, Fettalkoholethylenglycolether, Alkyloligoglucoside und Alkalimetallhydroxide enthalten. Schließlich offenbart die **WO 91/47760** ein schaumgedämpftes Maschinengeschirrspülmittel, das ausgesuchte Fettalkoholpolyglycolether zusammen mit Alkyloligoglucosiden enthält.

Bei Einsatz dieser bekannten Detergensmischungen in Handgeschirrspülmitteln müssen jedoch bislang in manchen Fällen Abstriche hinsichtlich des Leistungsvermögens und der ökotoxikologischen Verträglichkeit in Kauf genommen werden.

Die Aufgabe der Erfindung bestand somit darin, wäßrige Handgeschirrspülmittel auf der Basis von Alkyl- und/oder Alkenyloligoglykosiden mit weiter verbesserten Eigenschaften zu entwickeln. Ein besonderer Schwerpunkt hat vor allem darin bestanden, Konzentrate zur Verfügung zu stellen,
o die einen möglichst hohen Aktivsubstanzgehalt aufweisen,
o dabei flüssig oder zumindest fließfähig sind,
o einen möglichst niedrigen Kältetrübungspunkt besitzen,
o auch in konzentrierter Form dermatologisch unbedenklich, d. h. nicht hautreizend sind und
o gleichzeitig über ein hohes Tellerspülvermögen verfügen.

### Beschreibung der Erfindung

Gegenstand der Erfindung sind wäßrige Handgeschirrspülmittel, enthaltend
a) Alkyl- und/oder Alkenyloligoglykoside der Formel **(I)**,

   **R**^{**1**}**O-[G]**_{**p**} **(I)**

   in der R¹ für einen Alkylrest mit 8 bis 22 Kohlenstoffatomen, G für einen Zuckerrest mit 5 oder 6 Kohlenstoffatomen und p für Zahlen von 1 bis 10 steht;
b) Fettalkoholpolyglycolether der Formel **(II)**, 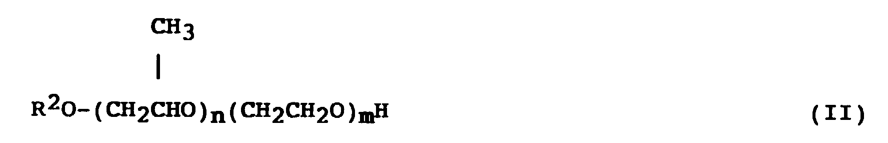 in der R² für einen Alkylrest mit 6 bis 14 Kohlenstoffatomen, n für Zahlen von 0,5 bis 2, und m für Zahlen von 2 bis 10 steht, und
c) amphotere bzw. zwitterionische Tenside.

Überraschenderweise wurde gefunden, daß die erfindungsgemäßen Mittel über ein Spül-, Schaum- und Reinigungsvermögen sowie eine hautkosmetische Verträglichkeit verfügen, die die der Einzelstoffe im Sinne einer synergistischen Verstärkung übertrifft. Gleichzeitig sind die Gemische auch in hochkonzentrierter Form von flüssig bzw. fließfähig und weisen Kältetrübunspunkte kleiner -3°C auf. Obschon Gemische von Alkyloligoglucosiden, Fettalkoholpolyglycolethern und Betainen aus dem Stand der Technik auch für die beanspruchte Verwendung grundsätzlich bekannt sind, können die erfindungsgemäßen Detergensgemische dennoch als nicht naheliegend angesehen werden, da der beanspruchte Kombinationseffekt von besonderer hautkosmetischer Verträglichkeit, hoher Spülleistung und vorteilhafter Rheologie nur innerhalb einer sehr engen Auswahl aus der Vielzahl technisch zur Verfügung stehender Fettalkoholpolyglycolether zu finden ist; die erfinderische Auswahl wird durch Vergleichbeispiele dokumentiert.

### Einsatzstoffe

**Alkyl- und/oder Alkenyloligoglykoside (APG)** stellen bekannte Stoffe dar, die nach den einschlägigen Verfahren der präparativen organischen Chemie erhalten werden können. Stellvertretend für das umfangreiche Schrifttum sei hier auf die Schriften **EP-A1-0 301 298** und **WO 90/3977** verwiesen.

Die Alkyl- und/oder Alkenyloligoglykoside können sich von Aldosen bzw. Ketosen mit 5 oder 6 Kohlenstoffatomen, vorzugsweise der Glucose ableiten. Die bevorzugten Alkyl- und/oder Alkenyloligoglykoside sind somit Alkyl- und/oder Alkenyloligo**glucoside**.

Die Indexzahl p in der allgemeinen Formel **(I)** gibt den Oligomerisierungsgrad (DP-Grad), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligoglykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt.

Detergensgemische mit besonders vorteilhaften Spüleigenschaften und optimaler dermatologischer Verträglich werden erhalten, wenn man kurzkettige und längerkettige Alkyl- und/oder Alkenyloligoglykoside, insbesondere aber Alkyloligoglucoside in geeigneter Weise kombiniert. Vorzugsweise setzt sich die Komponente a) zusammen aus
a1) Alkyloligoglucosiden der Formel **(III)**,

   **R**^{**3**}**O-[G]**_{**p1**} **(III)**

   in der R³ für einen Alkylrest mit 12 bis 16 Kohlenstoffatomen, G für einen Glucoserest und pl für Zahlen von 1 bis 3 steht, und
a2) Alkyloligoglucosiden der Formel **(IV)**,

   **R**^{**4**}**O-[G]**_{**p2**} **(IV)**

   in der R⁴ für einen Alkylrest mit 8 bis 11 Kohlenstoffatomen, G für einen Glucoserest und p2 für Zahlen von 1 bis 3 steht,
   wobei das Gewichtsverhältnis von a1) zu a2) im Bereich von 1 : 2 bis 2 : 1, vorzugsweise 1 : 0 bis 1 : 1 liegt.

Der Alkylrest R³ kann sich von primären Alkoholen mit 12 bis 16 Kohlenstoffatomen ableiten. Typische Beispiele sind Laurylalkohol, Myristylalkohol und Cetylalkohol sowie deren technische Gemische, die wie oben beschrieben erhalten werden können. Bevorzugt sind Alkyloligoglucoside auf Basis von gehärtetem C_{12/16}-Kokosalkohol mit einem DP von 1 bis 3.

Der Alkylrest R⁴ kann sich von primären Alkoholen mit 8 bis 11 Kohlenstoffatomen ableiten. Typische Beispiele sind Capronalkohol, Caprylalkohol und Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese anfallen. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂- Alkohol verunreinigt sein können sowie Alkyloligoglucoside auf Basis technischer C_{9/11}-Oxoalkohole (DP = 1 bis 3).

Die erfindungsgemäß einzusetzenden **Fettalkoholpolyglycolether** stellen Anlagerungsprodukte von Propylenoxid und Ethylenoxid an kurzkettige Fettalkohole dar. Typische Beispiele sind Addukte von durchschnittlich 0,5 bis 2 Mol Propylenoxid und 2 bis 10 Mol Ethylenoxid an Capronalkohol, Caprylalkohol, Caprinalkohol, Laurylalkohol und Myristylalkohol sowie deren technische Gemische, wie sie beispielsweise bei der Hochdruckhydrierung von technischen Kokos- oder Palmkernfettsäuremethylesterfraktionen anfallen. Vorzugsweise werdem Fettalkoholpolyglycolether der Formel **(II)** eingesetzt, in der R² für einen Alkylrest mit 8 bis 12 Kohlenstoffatomen, m für Zahlen von 1 bis 1,5 und n für Zahlen von 5 bis 9 steht. Die Produkte können eine konventionelle oder eingeengte Homologenverteilung aufweisen. Aus anwendungstechnischer Sicht sind ferner solche Fettalkoholpolyglycolether bevorzugt, die einen innenständigen Polypropylenglycol- und einen terminalen Polyethylenglycol-Block aufweisen und bei denen das Verhältnis EO : PO im Molekül größer/gleich 2 : 1 ist.

Als **amphotere bzw. zwitterionische Tenside** kommen beispielsweise Alkylbetaine, Alkylamidobetaine, Aminopropionate, Aminoglycinate, Imidazoliniumbetaine und/oder Sulfobetaine in Betracht.

Typische Beispiele sind die Umsetzungsprodukte von Fettaminen, Fettsäureamiden, Fettsäureaminoaminen oder Fettalkylimidazolinen mit Natriumchloracetat, Acrylsäureester oder Chlorhydroxypropansulfonsäure. Zu Herstellung und Struktur der genannten Verbindungen sei auf **Falbe (ed.), "Surfactants in consumer products", Springer-Verlag, 1986, S.114-119** verwiesen. Vorzugsweise werden Alkylamidobetaine eingesetzt, die durch Kondensation von technischer C_{8/14}- bzw. C_{8/18}-Kokosfettsäure mit Dimethylaminopropylamin und nachfolgende Reaktion mit Natriumchloracetat erhalten werden.

### Gewerbliche Anwendbarkeit

Die erfindungsgemäßen Mittel zeichnen sich durch ausgezeichnete Spül-, Schaum- und Reinigungsleistung sowie eine hohe hautkosmetische und ökotoxikologische Verträglichkeit aus. Wäßrige Handgeschirrspülmittel, die sich durch besonders vorteilhafte anwendungstechnische Eigenschaften auszeichnen, enthalten
15 bis 80 (20 bis 50) Gew.-% der Komponente a),
20 bis 50 (40 bis 50) Gew.-% der Komponente b),
20 bis 25 Gew.-% der Komponente c) und
mit der Maßgabe, daß sich die Anteile mit Wasser zu 100 Gew.-% ergänzen; in () angegeben befinden sich die besonders bevorzugten Konzentrationsbereiche. Wie oben angegeben, können die Detergensgemische einen Feststoffgehalt von 60 bis 100 Gew.-% aufweisen; Gemische mit besonders vorteilhaftem Fließverhalten werden jedoch im Bereich von 65 bis 70 Gew.-% Feststoff erhalten.

Wie oben beschrieben, haben sich folgende Detergensgemische als besonders vorteilhaft erwiesen, bei denen sich die Komponente a) aus kurzkettigen und längerkettigen Alkyloligoglucosiden zusammensetzt:
10 bis 80 (25 bis 70) Gew.-% der Komponente a1),
5 bis 40 (15 bis 20) Gew.-% der Komponente a2),
20 bis 50 (30 bis 40) Gew.-% der Komponente b) und
20 bis 25 Gew.-% der Komponente c),
ebenfalls mit der Maßgabe, daß sich die Anteile der Komponenten mit Wasser zu 100 Gew.-% ergänzen; in () angegeben befinden sich wiederum die besonders bevorzugten Konzentrationsbereiche.

Die Herstellung der Mittel kann durch einfaches mechanisches Vermischen der Einsatzstoffe, gegebenenfalls bei erhöhten Temperaturen von 30 bis 50°C erfolgen; eine chemische Reaktion findet hierbei nicht statt.

Neben den genannten Detergensgemischen können die erfindungsgemäßen Handgeschirrspülmittel weitere übliche Bestandteile, bei- spielsweise weitere anionische, nichtionische oder amphotere bzw. zwitterionische Cotenside, Schaumbooster, Duftstoffe etc. aufweisen. Eine besonders leistungsstarke und äußerst hautverträgliche Rezeptur kann beispielsweise 18 Gew.-% C₁₂/₁₆-APG, 10 Gew.-% C_{8/10}-APG, 26 Gew.-% Octanol-1,2PO-9EO- Addukt und 18 Gew.-% Alkylamidobetain (Wasser ad 100 Gew.-%) enthalten. Der Feststoffgehalt der Handgeschirrspülmittel kann 15 bis 50, vorzugsweise 20 bis 40 Gew.-% betragen.

### Beispiele

### I. Eingesetzte Tenside

A1. C_{12/16}-Kokosalkyloligoglucosid
   Plantaren^{(R)} APG 600 CS UP (50 Gew.-% AS in Wasser);
A2. C_{8/10}-Alkyloligoglucosid
   Plantaren^{(R)} APG 225 CS UP (68 Gew.-% AS in Wasser);
B1. Octanol-1,2PO-9EO-Addukt;
B2. Octanol-1,2-PO-Addukt;
B3. Octanol-9EO-Addukt;
B4. C_{12/14}-Kokosfettalkohol-7EO-Addukt;
C1. Betaintensid auf Basis C_{12/14}-Kokosfettsäureamid
   Dehyton^{(R)} K, gefriergetrocknet

Alle eingesetzten Tenside sind Verkaufsprodukte der Henkel KGaA, Düsseldorf/FRG. Die Stoffe B1 bis B4 und C1 sind wasserfrei.

### II. Beurteilung des Tellerspülvermögens (TSV)

Die Ermittlung des Tellerspülvermögens wurde mit Hilfe des Teller-Testes [**Fette, Seifen, Anstrichmitt., 74, 163 (1972)**] durchgeführt. Hierzu wurden Teller mit einem Durchmesser von 14 cm mit je 2 cm³ Rindertalg (Säurezahl 9-10) angeschmutzt und 24 h bei Raumtemperatur gelagert. Anschließend wurden die Teller bei 50°C mit 5 l Leitungswasser der Härte 16°d gespült. Die Prüfmischung wurde mit einer Dosierung von 0,15 g Aktivsubstanz/l eingesetzt. Der Spülversuch wurde abgebrochen, sobald der Schaum vollständig verschwunden war. Die Ergebnisse der Spülversuche sind in Tab.1 zusammengefaßt; angegeben ist die Anzahl gespülter Teller (GT).

**Tab.1:**

| Tellerspülvermögen | | | | | | | | | |
|---|---|---|---|---|---|---|---|---|---|
| Die Rezepturen enthalten 28 Gew.-% Wasser | | | | | | | | | |
| Bsp. | Zusammensetzung (Gew.-%) | | | | | | | GT Zahl | KTP °C |
| | A1 | A2 | B1 | B2 | B3 | B4 | C1 | | |
| 1 | 18 | - | 36 | - | - | - | 18 | 12/ 9 | <-3 |
| 2 | 18 | 10 | 26 | - | - | - | 18 | 13/10 | <-3 |
| V1 | 24 | - | - | 48 | - | - | - | 8/ 6 | 0 |
| V2 | 24 | - | - | - | 48 | - | - | 8/ 6 | -1 |
| V3 | 24 | - | - | - | - | 48 | - | 8/ 6 | -1 |
| V4 | 54 | - | - | - | - | - | 18 | 8/ 6 | -1 |
| V5 | 18 | - | - | 36 | - | - | 18 | 9/ 7 | -1 |
| V6 | 18 | 10 | - | 26 | - | - | 18 | 9/ 7 | -2 |
| Legende: GT = Anzahl gespülter Teller : fettfrei/mit Sprenkel KTB = Kältetrübungspunkt | | | | | | | | | |

## Patentansprüche

1. Wäßrige Handgeschirrspülmittel, enthaltend
a) Alkyl- und/oder Alkenyloligoglykoside der Formel **(I)**,
**R**^{**1**}**O-[G]**_{**p**} **(I)**
in der R¹ für einen Alkylrest mit 8 bis 22 Kohlenstoffatomen, G für einen Zuckerrest mit 5 oder 6 Kohlenstoffatomen und p für Zahlen von 1 bis 10 steht;
b) Fettalkoholpolyglycolether der Formel **(II)**, 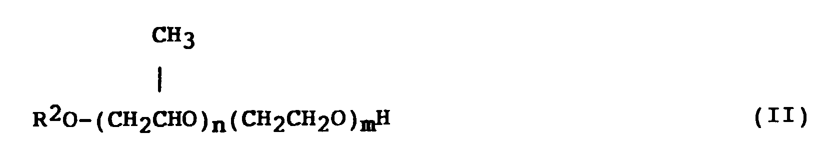 in der R² für einen Alkylrest mit 6 bis 14 Kohlenstoffatomen, n für Zahlen von 0,5 bis 2, und m für Zahlen von 2 bis 10 steht, und
c) amphotere bzw. zwitterionische Tenside.

2. Mittel nach Anspruch 1, **dadurch gekennzeichnet**, daß sich die Komponente a) zusammensetzt aus
a1) Alkyloligoglucosiden der Formel **(III)**,
**R**^{**3**}**O-[G]**_{**p1**} **(III)**
in der R³ für einen Alkylrest mit 12 bis 16 Kohlenstoffatomen, G für einen Glucoserest und p1 für Zahlen von 1 bis 3 steht, und
a2) Alkyloligoglucosiden der Formel **(IV)**,
**R**^{**4**}**O-[G]**_{**p2**} **(IV)**
in der R⁴ für einen Alkylrest mit 8 bis 11 Kohlenstoffatomen, G für einen Glucoserest und p2 für Zahlen von 1 bis 3 steht,
wobei das Gewichtsverhältnis von a1) zu a2) im Bereich von 1 : 2 bis 2 : 1 liegt.

3. Mittel nach den Ansprüchen 1 und 2, **dadurch gekennzeichnet**, daß sie Fettalkoholpolyglycolether der Formel **(II)** enthalten, in der R² für einen Alkylrest mit 8 bis 12 Kohlenstoffatomen, m für Zahlen von 1 bis 1,5 und n für Zahlen von 5 bis 9 steht.

4. Mittel nach den Ansprüchen 1 bis 3, **dadurch gekennzeichnet**, daß sie amphotere bzw. zwitterionische Tenside enthalten, ausgewählt aus der Gruppe, die gebildet wird von Alkylbetainen, Alkylamidobetainen, Aminopropionaten, Aminoglycinaten, Imidazoliniumbetainen und Sulfobetainen.

## Claims

1. Water-based manual dishwashing detergents containing
a) alkyl and/or alkenyl oligoglycosides corresponding to formula (I):
R¹O-[G]ₚ (I)
in which R¹ is an alkyl radical containing 8 to 22 carbon atoms, G is a sugar unit containing 5 or 6 carbon atoms and p is a number of 1 to 10;
b) fatty alcohol polyglycol ethers corresponding to formula (II): 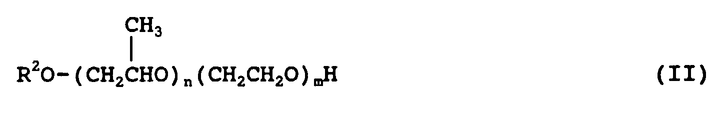 in which R² is an alkyl radical containing 6 to 14 carbon atoms, n is a number of 0.5 to 2 and m is a number of 2 to 10 and
c) amphoteric or zwitterionic surfactants.

2. Detergents as claimed in claim 1, characterized in that component a) consists of
a1) alkyl oligoglucosides corresponding to formula (III):
R³O-[G]ₚ₁ (III)
in which R³ is an alkyl radical containing 12 to 16 carbon atoms, G is a glucose unit and p1 is a number of 1 to 3, and
a2) alkyl oligoglucosides corresponding to formula (IV):
R⁴O-[G]ₚ₂ (IV)
in which R⁴ is an alkyl radical containing 8 to 11 carbon atoms, G is a glucose unit and p2 is a number of 1 to 3,
the ratio by weight of a1) to a2) being in the range from 1:2 to 2:1.

3. Detergents as claimed in claims 1 and 2, characterized in that they contain fatty alcohol polyglycol ethers corresponding to formula (II), in which R² is a C₈₋₁₂ alkyl radical, m is a number of 1 to 1.5 and n is a number of 5 to 9.

4. Detergents as claimed in claims 1 to 3, characterized in that they contain amphoteric or zwitterionic surfactants selected from the group consisting of alkyl betaines, alkyl amidobetaines, aminopropionates, aminoglycinates, imidazolinium betaines and sulfobetaines.

## Revendications

1. Produits aqueux de lavage de la vaisselle à la main, contenant :
a) des alkyl- et/ou alcényloligoglycosides de formule (I) :
R¹O-[G]ₚ (I)
où R¹ représente un radical alkyle de 8 à 22 atomes de carbone, G est un radical sucre de 5 ou 6 atomes de carbone et p représente des nombres de 1 à 10 ;
b) éther de polyglycol et d'alcool gras de formule (II) : 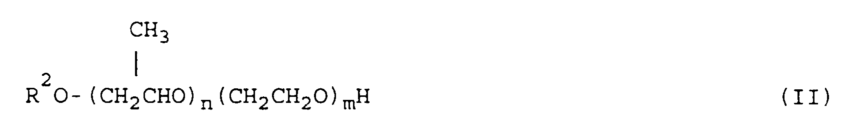 ou R² représente un radical alkyle de 6 à 14 atomes de carbone, n des nombres de 0,5 à 2 et m des nombres de 2 à 10, et
c) des tensioactifs amphotères ou zwitterioniques.

2. Produits selon la revendication 1,
caractérisé en ce que
le composant a) se compose des :
a1) alkyloligoglucosides de formule (III),
R³O-[G]ₚ₁ (III)
où R³ représente un radical alkyle de 12 à 16 atomes de carbone, G est un radical glucose et p, représente des nombres de 1 à 3, et
a2) des alkyloligoglucosides de formule (IV) :
R⁴O-[G]ₚ₂ (IV)
où R⁴ représente un radical alkyle de 8 à 11 atomes de carbone, G un radical glucose et p2 des nombres de 1 à 3 où le rapport pondéral de a1) à a2) est dans l'intervalle de 1:2 à 2:1.

3. Produits selon les revendications 1 et 2,
caractérisés en ce qu'
ils contiennent des éthers de polyglycol et d'alcools gras de formule (II), où R² représente un radical alkyle de 8 à 12 atomes de carbone, m représente des nombres de 1 à 1,5 et n des nombres de 5 à 9.

4. Produits selon les revendications 1 à 3,
caractérisés en ce qu'
ils contiennent des tensioactifs amphotères ou zwitterioniques, choisis dans le groupe formé par les alkylbétaïnes, alkylamidobétaïnes, aminopropionates, aminoglycinates, imidazoliniumbétaïnes et sulfobétaïnes.
